(19) Europäisches Patentamt
European Patent Office
Office européen des brevets

(11) Numéro de publication : **0 178 212**
**B1**

(12) **FASCICULE DE BREVET EUROPÉEN**

(45) Date de publication du fascicule du brevet : **28.09.88**

(51) Int. Cl.⁴ : **A 61 K 47/00**

(21) Numéro de dépôt : **85401834.8**

(22) Date de dépôt : **20.09.85**

# (54) Véhicule pharmaceutique pour substances actives se présentant sous forme d'un gel anhydre.

(30) Priorité : **21.09.84 FR 8414512**

(43) Date de publication de la demande :
**16.04.86 Bulletin 86/16**

(45) Mention de la délivrance du brevet :
**28.09.88 Bulletin 88/39**

(84) Etats contractants désignés :
**BE CH DE GB IT LI NL SE**

(56) Documents cités :
**EP-A- 0 076 068**
**GB-A- 2 107 588**
**US-A- 3 946 106**
**CHEMICAL ABSTRACTS, vol. 100, no. 24, juin 1984, page 369, no. 197819m, Columbus, Ohio, US**

(73) Titulaire : **CENTRE INTERNATIONAL DE RECHERCHES DERMATOLOGIQUES C.I.R.D. Groupement d'Intérêt Economique dit:**
**Sophia Antipolis**
**F-06560 Valbonne (FR)**

(72) Inventeur : **Caron, Danièle**
**3, rue de la Boyère Haut Sartoux**
**F-06560 Valbonne (FR)**
Inventeur : **Shroot, Braham Villa 35**
**Hameaux de Val-Bosquet Chemin de Val-Bosquet**
**F-06600 Antibes (FR)**

(74) Mandataire : **Nony, Michel**
**Cabinet Nony 29, rue Cambacérès**
**F-75008 Paris (FR)**

**EP 0 178 212 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un nouveau véhicule pharmaceutique pour substances actives, notamment pour des substances sensibles à l'oxydation, se présentant sous forme d'un gel anhydre.

De nombreuses substances actives sensibles à l'oxydation par l'oxygène de l'air sont utilisées en thérapeutique dans des traitements dermatologiques et l'on peut citer à cet égard l'anthraline ou dithranol et ses dérivés, l'idoxuridine, la trétinoïne, etc.

Parmi ces substances, l'anthraline est un composé particulièrement actif dans le traitement du psoriasis mais il présente l'inconvénient d'être très facilement dégradé par oxydation en quinones ou en produits polymériques, de couleur foncée, susceptibles de tâcher non seulement la peau mais également les vêtements.

Diverses formes de compositions ont déjà été proposées en vue du conditionnement et de l'administration de substances instables ou facilement oxydables telle que l'anthraline.

Dans le brevet belge n° 894.778 on propose notamment des compositions épaissies constituées d'au moins un alkyl ester d'acide gras, l'acide gras ayant de 12 à 18 atomes de carbone et le radical alkyle, ramifié ou non, ayant 2 ou 3 atomes de carbone, et d'au moins un agent épaississant pris dans le groupe constitué par les silices, ayant une granulométrie inférieure à 30 m et les poudres de polyéthylènes ayant une masse volumique comprise entre 0,9 et 0,96 ($g/cm^3$).

Les compositions de ce brevet permettent de stabiliser l'anthraline et ses dérivés mais l'on a constaté que ce type de véhicule ne favorisait pas une pénétration satisfaisante de l'anthraline mais plutôt sa concentration dans les couches supérieures de la peau.

Par ailleurs, les compositions doivent présenter une viscosité suffisante permettant d'éviter tout écoulement sur les parties saines sous peine d'entraîner éventuellement des phénomènes d'irritation de la peau.

Les compositions utilisant comme agent épaississant des silices ou des résines du type polyéthylène, si elles présentent une viscosité appropriée, s'éliminent parfois difficilement, ce qui par ailleurs constitue un inconvénient, indépendamment du fait que l'on n'obtient pas toujours une bonne pénétration.

La présente invention concerne un nouveau véhicule pharmaceutique se présentant sous forme d'un gel anhydre permettant non seulement une bonne conservation des substances actives instables, oxydables, mais facilitant également une bonne disponibilité des substances actives incorporées.

La présente invention a pour objet à titre de produit industriel nouveau, un véhicule pharmaceutique pour substances actives se présentant sous forme d'un gel anhydre, ayant une viscosité comprise entre 540 et 580 cps (CONTRAVES) ledit gel étant essentiellement constitué d'huile de paraffine, d'au moins un alkyl ester d'acide gras et d'un silicone élastomère du type polyvinyldiméthyl siloxane en tant qu'agent épaississant.

Les différents essais réalisés ont permis de mettre en évidence l'importance du choix des trois ingrédients principaux du gel selon l'invention.

Ainsi, lorsque l'on cherche à remplacer l'huile de paraffine par d'autres produits huileux ou le silicone élastomère du type polyvinyldiméthyl siloxane par d'autres substances, on a observé un manque de stabilité des compositions et/ou une diminution notable de la pénétration des composés actifs et/ou une viscosité ne répondant pas aux critères requis.

Le nouveau véhicule selon l'invention trouve donc non seulement une application à l'égard de substances facilement oxydables telles que l'anthraline mais également vis à vis d'autres substances pour lesquelles on recherche une bonne pénétration en vue d'en accroître l'activité.

A cet égard, le nouveau véhicule selon l'invention est particulièrement approprié pour des produits actifs à action locale tels que par exemple certains agents anti-inflammatoires comme l'hydrocortisone et anti-acnéiques comme les rétinoïdes.

L'agent épaississant du gel anhydre selon l'invention est un silicone élastomère du type polyvinyldiméthylsiloxane associé à une charge de silice.

Les silicones élastomères du véhicule selon l'invention peuvent être représentés par la formule suivante:

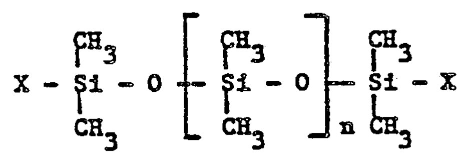

dans laquelle: X représente OH et n est tel que la viscosité du silicone élastomère associé à la charge de silice soit comprise entre environ 410 et 480 ps mesurée à 24 °C (CONTRAVES).

Cet agent épaississant présente une bonne compatibilité avec les deux autres ingrédients du gel et permet d'obtenir la viscosité recherchée.

De tels produits sont notamment décrits dans le BSM 8424 M.

Comme silicone élastomère préféré, on peut en particulier mentionner le produit vendu par la société DOW CORNING CORPORATION sous la dénomination de « DOW CORNING MDX-4-4210 » qui se présente sous forme d'un kit constitué d'une part d'un produit de base (silicone élastomère + silice) et d'autre part d'un agent de réticulation ou catalyseur de vulcanisation, le produit de base, de viscosité d'environ 450 ps (CONTRAVES), étant seul utilisé selon la présente invention pour constituer l'agent épaississant.

L'alkyl ester d'acide gras est un ester d'un acide gras ayant de 12 à 18 atomes de carbone, le radical alkyle, linéaire ou ramifié, ayant 2 ou 3 atomes de carbone.

Parmi les alkyl esters d'acides gras préférés, on peut citer le myristate d'isopropyle, le palmitate d'isopropyle, le myristate d'éthyle, et leurs mélanges.

L'huile de paraffine liquide sert à réfléchir la lumière, à faciliter la pénétration et à donner un bon aspect au gel.

Afin d'obtenir la viscosité requise, le rapport en poids du silicone élastomère à l'alkyl ester d'acide gras doit de préférence être compris entre 0,90 et 1,25.

Selon une forme de réalisation préférée, le véhicule pharmaceutique sous forme de gel anhydre est constitué de:

0,5 à 2 % en poids d'huile de paraffine liquide,
50 ± 2,5 % en poids d'alkyl ester(s) d'acide gras, et
49 ± 2,5 % en poids de silicone élastomère de type polyvinyldiméthylsiloxane.

Les substances actives qui doivent être administrées à l'aide du gel anhydre selon l'invention, sont généralement présentes à une concentration comprise entre 0,05 et 5 %, ces substances pouvant être soit solubles, soit partiellement solubles dans le gel.

Parmi les substances actives administrables à l'aide du gel selon l'invention on peut tout particulièrement mentionner, l'anthraline, les acides cis et trans-rétinoïques, l'hydrocortisone, le 17-α-butyrate d'hydrocortisone, etc.

Bien que les exemples suivants se réfèrent à ces différentes substances actives, il va de soi que le véhicule pharmaceutique selon l'invention n'est pas pour autant limité à ces substances et peut également convenir pour d'autres substances actives pour lesquelles l'on recherche une bonne conservation et/ou une bonne pénétration.

Exemple 1: Gel à base d'anthraline

Ce gel est obtenu en procédant au mélange des constituants suivants:

| | |
|---|---|
| myristate d'isopropyle | 49,70 g |
| silicone élastomère « DOW CORNING MDX-4-4210 » | 49,00 g |
| huile de paraffine liquide | 1,00 g |
| anthraline | 0,30 g |

Exemple 2: Gel à base d'acide All-trans-rétinoïque

Ce gel est obtenu en procédant au mélange des constituants suivants:

| | |
|---|---|
| myristate d'isopropyle | 49,95 g |
| silicone élastomère « DOW CORNING MDX-4-4210 » | 49,00 g |
| huile de paraffine liquide | 1,00 g |
| acide all-trans-rétinoïque | 0,05 g |

Exemple 3: Gel à base de 17-α-butyrate d'hydrocortisone

Ce gel est obtenu en procédant au mélange des constituants suivants:

| | |
|---|---|
| palmitate d'isopropyle | 49,90 g |
| silicone élastomère « DOW CORNING MDX-4-4210 » | 49,00 g |
| huile de paraffine liquide | 1,00 g |
| 17-α-butyrate d'hydrocortisone | 0,10 g |

Exemple 4: Gel à base d'hydrocortisone

Ce gel est obtenu en procédant au mélange des constituants suivants:

| | |
|---|---|
| myristate d'isopropyle | 49,90 g |
| silicone élastomère « DOW CORNING MDX-4-4210 » | 49,00 g |
| huile de paraffine liquide | 1,00 g |
| hydrocortisone | 0,10 g |

**Revendications**

1. Véhicule pharmaceutique pour substances actives, caractérisé par le fait qu'il se présente sous forme d'un gel anhydre ayant une viscosité comprise entre 540 et 580 cps, ledit gel étant essentiellement constitué d'huile de paraffine, d'au moins un alkyl ester d'acide gras et d'un silicone élastomère du type polyvinyldiméthylsiloxane en tant qu'agent épaississant.

2. Véhicule selon la revendication 1, caractérisé par le fait que le silicone élastomère du type polyvinyldiméthylsiloxane est associé à une charge de silice et correspond à la formule suivante:

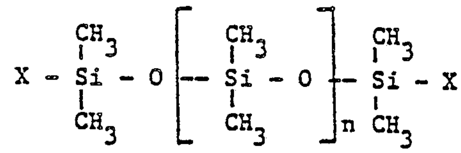

dans laquelle: X représente OH et n est tel que la viscosité du silicone élastomère associée à la charge de silice soit comprise entre environ 410 et 480 cps mesurée à 24 °C.

3. Véhicule selon l'une quelconque des revendications 1 et 2, caractérisé par le fait que l'alkyl ester d'acide gras est un ester d'un acide gras ayant de 12 à 18 atomes de carbone, le radical alkyle, linéaire ou ramifié, ayant 2 ou 3 atomes de carbone.

4. Véhicule selon la revendication 3, caractérisé par le fait que l'alkyl ester d'acide gras est le myristate d'isopropyle, le palmitate d'isopropyle, le myristate d'éthyle, et leurs mélanges.

5. Véhicule selon l'une quelconque des revendications précédentes, caractérisé par le fait que le rapport en poids du silicone élastomère à l'alkyl ester d'acide gras est compris entre 0,90 et 1,25.

6. Véhicule selon l'une quelconque des revendications précédentes, caractérisé par le fait qu'il est constitué de:

0,5 à 2 % en poids d'huile de paraffine liquide,

50 ± 2,5 % en poids d'alkyl ester d'acide gras, et

49 ± 2,5 % en poids de silicone élastomère de type polyvinyldiméthylsiloxane.

7. Application d'un véhicule selon l'une quelconque des revendications précédentes, caractérisée par le fait qu'une substance active est présente dans ledit véhicule, à une concentration comprise entre 0,05 et 5 % en poids par rapport au poids total du gel anhydre.

8. Application selon la revendication 7, caractérisée par le fait que la substance active est l'anthraline, les acides cis et trans-rétinoïques, l'hydrocortisone ou le 17-α-butyrate d'hydrocortisone.

**Claims**

1. Pharmaceutical vehicle for active substances, characterized in that it takes the form of an anhydrous gel having a viscosity of between 540 and 580 cps, the said gel consisting essentially of liquid paraffin, at least one fatty acid alkyl ester and a silicone elastomer of the polyvinyldimethylsiloxane type as a thickening agent.

2. Vehicle according to Claim 1, characterized in that the silicone elastomer of the polyvinyldimethylsiloxane type is combined with a silica filler and corresponds to the following formula:

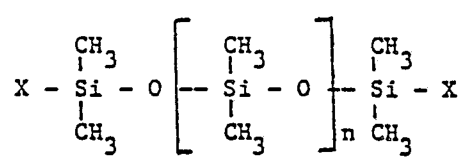

in which : X denotes OH and n is such that the viscosity of the silicone elastomer combined with the silica filler is between approximately 410 and 480 cps measured at 24 °C.

3. Vehicle according to either of Claims 1 and 2, characterized in that the fatty acid alkyl ester is an ester of a fatty acid having from 12 to 18 carbon atoms, the alkyl radical, linear or branched, having 2 or 3 carbon atoms.

4. Vehicle according to Claim 3, characterized in that the fatty acid alkyl ester is isopropyl myristate, isopropyl palmitate, ethyl myristate and mixtures thereof.

5. Vehicle according to any one of the preceding claims, characterized in that the ratio by weight of the silicone elastomer to the fatty acid alkyl ester is between 0.90 and 1.25.

6. Vehicle according to any one of the preceding claims, characterized in that it consists of:

0.5 to 2 % by weight of liquid paraffin,

50 ± 2,5 % by weight of fatty acid alkyl ester, and

49 ± 2.5 % by weight of silicone elastomer of the polyvinyldimethylsiloxane type.

7. Application of a vehicle according to any one of the preceding claims, characterized in that an active substance is present in the said vehicle at a concentration of between 0.05 and 5 % by weight, relative to the total weight of the anhydrous gel.

8. Application according to Claim 7, characterized in that the active substance is anthralin, cis- and trans-retinoic acids, hydrocortisone or hydrocortisone 17-α-butyrate.

## Patentansprüche

1. Pharmazeutischer Träger für aktive Substanzen, dadurch gekennzeichnet, daß er in Form eines wasserfreien Gels mit einer Viskosität zwischen 540 und 580 cps vorliegt, wobei dieses Gel im wesentlichen aus Paraffinöl, mindestens einem Fettsäurealkylester und einem elastomeren Silicon des Polyvinyldimethylsiloxan-Typs sowie einem Verdickungsmittel besteht.

2. Träger nach Anspruch 1, dadurch gekennzeichnet, daß das elastomere Silicon des Polyvinyldimethylsiloxan-Typs in Verbindung mit einem Silicafüllstoff vorliegt und folgender Formel entspricht:

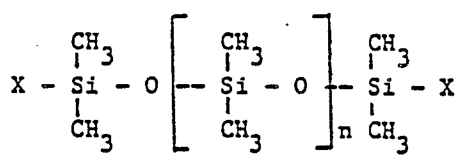

worin X für OH steht und n so beschaffen ist, daß die Viskosität des in Verbindung mit einem Silicafüllstoff vorliegenden elastomeren Silicons zwischens ca. 410 und 480 cps, gemessen bei 24 °C, beträgt.

3. Träger nach einem der Ansprüche 1 und 2, dadurch gekennzeichnet, daß der Fettsäurealkylester ein Ester einer Fettsäure mit 12 bis 18 Kohlenstoffatomen ist und der lineare oder verzweigte Alkylrest 2 oder 3 Kohlenstoffatome aufweist.

4. Träger nach Anspruch 3, dadurch gekennzeichnet, daß der Fettsäurealkylester i-Propylmyristat, i-Propylpalmitat, Ethylmyristat oder eine Mischung davon ist.

5. Träger nach einem der vorhergehenden Ansprüche, dadurch gekennzeichnet, daß das Gewichtsverhältnis des elastomeren Silicons zum Fettsäurealkylester zwischen 0,90 und 1,25 liegt.

6. Träger nach einem der vorhergehenden Ansprüche dadurch gekennzeichnet, daß er aus

0,5 bis 2 Gew.-% flüssigem Paraffinöl,

50 + 2,5 Gew.-% Fettsäurealkylester und

49 + 2,5 Gew.-% elastomeren Silicon des Polyvinyldimethylsiloxan-Typs besteht.

7. Verwendung eines Trägers nach einem der vorhergehenden Ansprüche, dadurch gekennzeichnet, daß eine aktive Substanz in einer Konzentration zwischen 0,05 und 5 Gew.-%, bezogen auf das Gesamtgewicht des wasserfreien Gels, im Träger vorhanden ist.

8. Verwendung nach Anspruch 7, dadurch gekennzeichnet, daß die aktive Substanz Anthralin, cis- und trans-Retinoesäure, Hydrocortison oder das Hydrocortison-17-α-butyrat ist.